# EUROPEAN PATENT APPLICATION

(11) **EP 3 903 937 A1**
(43) Date of publication of application: **03.11.2021**
(21) Application number: 19905038.6
(22) Date of filing: 30.12.2019
(51) Int. Cl.: B01J 38/58, B01J 38/52, B01J 23/62, B01J 23/96, C07C 29/147

(54) **METHOD FOR REGENERATING HYDROGENATION CATALYST**

(30) Priority: 28.12.2018 KR 20180173038
(71) Applicant: Hanwha Solutions Corporation, Jung-gu Seoul 04541 (KR)
(72) Inventor: YOOK, Sun Woo, Daejeon 34128 (KR); KIM, Jeong Kwon, Daejeon 34128 (KR); JEON, Bong Sik, Daejeon 34128 (KR); MYEONG, Wan Jae, Daejeon 34128 (KR)
(74) Representative: Klunker IP Patentanwälte PartG mbB
(86) International application number: PCT/KR2019/017330
(87) International publication number: WO 2020/138762

(57) **Abstract**

The present invention relates to a method for regenerating a dicarboxylic acid or carboxylic acid hydrogenation catalyst, and more particularly, to a method for regenerating a hydrogenation catalyst to be used in a reaction of converting a dicarboxylic acid group into a diol group. The present invention provides an effect of regenerating a catalyst deactivated by the deposition of esters to be produced in a reaction of converting a dicarboxylic acid group into a diol group.

## Description

### TECHNICAL FIELD

The present invention relates to a method for regenerating a hydrogenation catalyst to be used in a reaction of converting a dicarboxylic acid group into a diol group. The present invention provides a method for regenerating a catalyst through washing of a catalyst using an organic solvent and a hydrothermal hydrogenation reaction.

### BACKGROUND ART

Diol is widely used as a base industrial material such as polyesters, polyurethanes, vanishes, and adhesives pharmaceuticals. In addition, the demand for eco-friendly and biodegradable diols has increased significantly in recent years. Among a plurality of diol compounds, when cyclohexanedimethanol (CHDM) is used to replace ethylene glycol and other polyols in producing polyester resins, the CHDM has attracted great attention because of its high thermal stability, insulation, transparency, and chemical resistance.

These diol compounds can be produced from dicarboxylic acid or derivatives thereof through a hydrodeoxygenation reaction using a hydrogenation catalyst (hydrogenation catalyst). Most existing catalyst processes that enable this are catalyst processes based on ruthenium-tin-carbon complex, ruthenium-carbon complex, ruthenium oxide, Cu-Cr, or Zr-Cr. However, such catalyst processes require a reaction at high temperature (150-300°C) and high pressure (100-300 bar).

Japanese Patent Registration No. 3726504, which is Mitsubishi Chemical's registered patent, discloses a method for regenerating a catalyst whose activity is reduced by using it for hydrogenation of carboxylic acid and carboxylic acid ester. As the method for regenerating the catalyst, a catalyst having reduced activity is treated with a base. However, if the base is used, there is a problem that Sn is eluted in the form of sodium stannate.

U.S. Patent No. 4533648, which is The Procter & Gamble Company's registered patent, discloses a high-temperature vacuum and oxidation treatment for reducing the content of organic materials remaining in the catalyst as a method for regenerating a Cu-Cr catalyst used for hydrogenation of carboxylic acid or carboxylic acid ester. However, a carbon-containing catalyst has a limitation in that there is a possibility that the catalyst can be burned under the oxidation treatment conditions.

Korean Patent Registration No. 2009-0031793, which is Johnson Matthey Davy Technologies Limited's published patent document, discloses a ruthenium/phosphine homogeneous catalyst, which can be used as a hydrogenation catalyst for carboxylic acid or derivatives thereof, and a method for regenerating the same. The known regeneration method in the published patent document is to react a catalyst in the presence of hydrogen and water. At this time, it has been reported that the catalyst poisoned by carbon monoxide reacted with water and hydrogen to convert carbon monoxide into carbon dioxide and methane, and the activity of the catalyst was revived. However, in this case, a homogeneous catalyst was used, and it cannot be said that the cause of catalyst deactivation (catalyst deposition by esters and organic materials) occurring in the existing heterogeneous catalyst is solved.

Korean Patent Registration No. 10-2012-0056040, which is LG Chem's registered patent, discloses a method for regenerating a hydrogenation catalyst for alcohol production, wherein hydrogen gas is flowed under high temperature and normal pressure. However, even in this case, there is a limitation in that the catalyst regeneration process aims to regenerate a catalyst poisoned by a material such as phosphorus or acid.

Therefore, there is a need for a method capable of regenerating a hydrogenation catalyst deactivated by fouling in hydrogenation of carboxylic acid or carboxylic acid ester.
(Patent Literature 1) Japanese Patent Registration No. 3726504 (2005. 10. 07.)
(Patent Literature 2) U.S. Patent No. 4533648 (1985. 08. 06.)
(Patent Literature 3) Korean Patent Application Publication No. 2009-0031793 (2009. 03.27.)
(Patent Literature 4) Korean Patent Registration No. 10-2012-0056040 (2014. 08. 06.)

### DESCRIPTION OF EMBODIMENTS

### TECHNICAL PROBLEM

The present invention aims to solve the above-described problems of the related art and the technical problems requested from the past.

An object of the present invention is to provide a method for regenerating a catalyst for a hydrogenation reaction of carboxylic acid or carboxylic acid ester by using an organic solvent.

In addition, an object of the present invention is to provide a method for regenerating a catalyst for a hydrogenation reaction of carboxylic acid or carboxylic acid ester by using a hydrothermal hydrogenation reaction.

In particular, an object of the present invention is to provide a relatively simple regeneration method in the case of using an organic solvent, and the regeneration methods aim to restore the activity of the catalyst to that similar to initial activity.

In particular, an object of the present invention is to regenerate the activity of the catalyst by removing catalyst fouling caused by esters or the like produced in the conversion reaction of dicarboxylic acid to a diol group.

### SOLUTION TO PROBLEM

In order to achieve the above objects, the present invention provides a method for regenerating a catalyst by using an organic solvent and a method for regenerating a catalyst by using a hydrothermal hydrogenation reaction as a method for regenerating a catalyst used in a hydrogenation reaction of carboxylic acid or carboxylic acid ester.

A method for regenerating a catalyst by using an organic solvent includes the steps of: (a) adding a used catalyst to an organic solvent and washing the catalyst while stirring the catalyst; (b) separating and recovering the catalyst by filtering after the washing; and (c) drying and reactivating the separated and recovered catalyst.

The organic solvent may include at least one selected from the group consisting of acetone, pyridine, hexafluoroisopropanol, methanol, ethanol, propanol, butanol, cyclohexane, toluene, and dichloromethane.

The step (a) of washing the catalyst while stirring the catalyst may be performed at 0-150°C, preferably room temperature, for 0.15-12 hours. This step may be performed once or more.

The drying in the step (c) may be performed at a temperature of 40-200°C for 1-24 hours, and may be the pressure during the drying may be 0-1 bar.

On the other hand, a method for regenerating a catalyst using a hydrothermal hydrogenation reaction includes the steps of: (i) adding a used catalyst and a solvent to a reactor; (ii) replacing a composition of gas inside the reactor with hydrogen; and (iii) reactivating the catalyst by performing a hydrothermal hydrogenation reaction while stirring under a condition in which a reactor internal temperature is 100-400°C and a hydrogen gas pressure is 1-20 MPa.

In addition, the hydrothermal hydrogenation reaction in the step (iii) may be performed for 0.5-48 hours, and preferably 12 hours.

The hydrothermal hydrogenation reaction in the step (iii) may be more preferably performed at 200-300°C, and more. The hydrothermal hydrogenation reaction in the step (iii) may be more preferably performed at 3-15 MPa.

According to an embodiment of the present invention, the hydrogenation reaction, which uses the catalyst, may convert a carboxylic acid functional group, a carboxylic acid ester functional group, an aldehyde functional group, or a ketone functional group into an alcohol functional group.

In particular, the carboxylic acid functional function may convert a dicarboxylic acid group into a diol group.

In addition, according to an embodiment of the present invention, the activity of the used catalyst may be lowered by fouling caused by deposition of a material produced in a process of converting a dicarboxylic acid group into a diol group. The deposited produced material may be preferably ester.

According to an embodiment of the present invention, the deactivated catalyst may include a precious metal-transition metal supported on a support.

The precious metal may include at least one selected from the group consisting of palladium (Pd), rhodium (Rh), ruthenium (Ru), iridium (Ir), and platinum (Pt), and the transition metal may include at least one selected from the group consisting of tin (Sn), iron (Fe), rhenium (Re), and gallium (Ga).

Furthermore, the support may include at least one selected from silica, alumina, zirconia, titania, and carbon. The carbon may be at least one selected from the group consisting of activated carbon, carbon black, graphite, graphene, ordered mesoporous carbon (OMC), and carbon nanotubes.

According to an embodiment of the present invention, the hydrogenation reaction pressure may be 1-20 MPa, the reaction temperature may be 100-400°C, and the reaction time may be 0.5-48 hours.

According to an embodiment of the present invention, the carboxylic acid may include one selected from the group consisting of oxalic acid, malonic acid, succinic acid, glutaric acid, adipic acid, pimelic acid, suberic acid, azelaic acid, sebacic acid, phthalic acid, isopthalic acid, cyclohexane dicarboxylic acid, and terephthalic acid.

According to an embodiment of the present invention, the aldehyde functional group may include one selected from the group consisting of formaldehyde, propionaldehyde, n-butylaldehyde, isobutylaldehyde, valeraldehyde, 2-methylbutylaldehyde, 3-methylbutylaldehyde, 2,2-dimethylpropionaldehyde, capronaldehyde, 2-methylvaleraldehyde, 3-methylvaleraldehyde, 4-methylvaleraldehyde, 2-ethylbutylaldehyde, 2,2-dimethylbutylaldehyde, 3,3-dimethylbutylaldehyde, caprylaldehyde, caprinealdehyde, and glutalaldehyde.

According to an embodiment of the present invention, the ketone functional group may include one selected from the group consisting of acetone, butanone, pentanone, hexanone, cyclohexanone, and acetophenone.

### ADVANTAGEOUS EFFECTS OF DISCLOSURE

As described above, according to the present invention, provided is a method for regenerating a catalyst used in a hydrogenation reaction for carboxylic acid or carboxylic acid ester, wherein the catalyst is washed using an organic solvent.

This aims to restore the activity of the catalyst to that similar to initial activity, while providing a relatively simple regeneration method.

In addition, according to the present invention, provided is a method for regenerating a catalyst used in a hydrogenation reaction of carboxylic acid or carboxylic acid ester by using a hydrothermal hydrogenation reaction, and the use of the method provides an effect of restoring the activity of the catalyst to that similar to initial activity.

In particular, the present invention has an effect of restoring the activity of the catalyst by removing catalyst fouling caused by esters produced in a reaction of converting dicarboxylic acid into a diol group.

Therefore, the regenerated catalyst can be reused to produce eco-friendly and biodegradable diols.

### BRIEF DESCRIPTION OF DRAWINGS

FIG. 1 is a result showing reaction activity of a deactivated hydrogenation catalyst after washing using an organic solvent.
FIG. 2 is a result showing reaction activity after hydrothermal hydrogenation treatment of a deactivated hydrogenation catalyst.
FIG. 3 is a result showing reaction activity of a deactivated hydrogenation catalyst after washing using a basic aqueous solution.
FIG. 4 is a result of STEM and EDX analysis of a ruthenium (Ru)-tin (Sn)/C catalyst washed using a basic aqueous solution (NaOH, NH₃).
FIG. 5 is a result of STEM and XRD analysis of a catalyst re-reduced while flowing hydrogen at 500°C.

### BEST MODE

The present invention will be described with reference to specific embodiments and the accompanying drawings. The embodiments will be described in detail in such a manner that the present invention may be carried out by those of ordinary skill in the art. It should be understood that various embodiments of the present invention are different, but need not be mutually exclusive. For example, certain shapes, structures, and features described herein may be implemented in other embodiments without departing from the spirit and scope of the present invention in connection with one embodiment.

Therefore, the following detailed description is not to be taken in a limiting sense, and the scope of the present invention is to be limited only by the appended claims and the entire scope of equivalents thereof, if properly explained.

In addition, unless otherwise specified in the present specification, the term "substitution" or "substituted" means that one or more hydrogen atoms in the functional groups of the present invention are substituted with one or more substituents selected from the group consisting of a halogen atom (-F, -Cl, -Br, or -I), a hydroxy group, a nitro group, a cyano group, an amino group, an amidino group, a hydrazine group, a hydrazone group, a carboxyl group, an ester group, a ketone group, a substituted or unsubstituted alkyl group, a substituted or unsubstituted alicyclic organic group, a substituted or unsubstituted aryl group, a substituted or unsubstituted alkenyl group, a substituted or unsubstituted alkynyl group, a substituted or unsubstituted heteroaryl group, and a substituted or unsubstituted heterocyclic group. These substituents may be linked to each other to form a ring.

In the present invention, unless otherwise specified, the term "substituted" means that a hydrogen atom is substituted with a substituent such as a halogen atom, a C₁-C₂₀ hydrocarbon group, a C₁-C₂₀ alkoxy group, and a C₆-C₂₀ aryloxy group.

In addition, unless otherwise specified, the term "hydrocarbon group" refers to a linear, branched, or cyclic saturated or unsaturated hydrocarbon group. The alkyl group, the alkenyl group, the alkynyl group, and the like may be linear, branched, or cyclic.

In addition, unless otherwise specified in the present specification, the term "alkyl group" refers to a C₁-C₃₀ alkyl group and the term "aryl group" refers to a C₆-C₃₀ aryl group. In the present specification, the term "heterocyclic group" refers to a group in which one to three heteroatoms selected from the group consisting of O, S, N, P, Si, and any combination thereof are contained in one ring. Examples of the heterocyclic group may include pyridine, thiophene, and pyrazine, but the present invention is not limited thereto.

In the detailed description of the present invention, the term "dicarboxylic acid" refers to an organic acid having two carboxylic acid functional groups in one molecule. For example, the molecular formula of the dicarboxylic acid is HOOC-R-COOH. In the present invention, R is preferably an alkyl group or an aryl group.

Hereinafter, embodiments of the present invention will be described with reference to the accompanying drawings, so that those of ordinary skill in the art can easily carry out the present invention.

According to an embodiment of the present invention, a method for regenerating a catalyst used in a hydrogenation reaction of carboxylic acid or carboxylic acid ester is provided. Specifically, a method for regenerating a catalyst by using an organic solvent and a method for regenerating a catalyst by using a hydrothermal hydrogenation reaction are provided.

First, the method for regenerating a catalyst by using an organic solvent includes the steps of: (a) adding a used catalyst to an organic solvent and washing the catalyst while stirring the catalyst; (b) separating and recovering the catalyst by filtering after the washing; and (c) drying and reactivating the separated and recovered catalyst.

The organic solvent may include at least one selected from the group consisting of acetone, pyridine, hexafluoroisopropanol, methanol, ethanol, propanol, butanol, cyclohexane, toluene, and dichloromethane. Preferably, acetone, pyridine, and hexafluoroisopropanol are provided. In this case, a deactivated catalyst may be included in an amount of 1-20 parts by weight based on 100 parts by weight of the organic solvent. Preferably, the deactivated catalyst may be included in an amount of 0.5-5 parts by weight. Organic materials that inhibit activation by causing fouling of the catalyst may be removed by sufficiently stirring and washing the catalyst within the above range.

In addition, the step (a) of washing the catalyst while stirring the catalyst may be performed at 0-150°C for 0.15-12 hours. Preferably, the step (a) of washing the catalyst while stirring the catalyst may be performed at room temperature for 1-3 hours.

When the temperature the step (a) of washing the catalyst while stirring the catalyst is less than 0°C, it is difficult to effectively remove organic materials (esters) that cause fouling. When the temperature the step (a) of washing the catalyst while stirring the catalyst is 150°C or higher, evaporation of the washing organic solvent easily occurs. Therefore, these ranges are not preferable.

A stirring speed of 100-1,000 rpm, preferably 300 rpm, is provided using a high-speed magnetic stirrer.

In addition, the step of washing the catalyst while stirring the catalyst may be provided once, and furthermore, may be performed twice or more. When the number of washing increases, the material fouled in the catalyst may be removed more smoothly, and thus it can further contribute to restoring the activity of the initial catalyst. However, when the washing is performed more than necessary, it is unreasonable in terms of time or cost. Therefore, preferably, the washing is performed 2-4 times.

However, the method for washing the catalyst as described above is not limited thereto, and the amount, type, number of times, etc. of the catalyst may be slightly modified at the level of those of ordinary skill in the art as necessary.

According to an embodiment of the present invention, after the washing is completed, the step of separating and recovering the catalyst by filtering is performed. In the filtering step, a vacuum pump or an aspirator may be used, and a membrane filer or a paper filter may be used to minimize the loss of catalyst. At this time, the filter may have a pore size of 0.5-5 um. However, an apparatus and a method used for the filtering are not limited thereto, and may be appropriately modified according to the amount of the catalyst to be filtered.

The separated and recovered catalyst may be dried and reused. The drying in the step (c) may be performed at 40-200°C for 2-24 hours. Preferably, the drying in the step (c) may be performed at 100°C for 8-12 hours. A vacuum oven may be used for faster drying. A pressure at this time may be preferably 0-0.5 bar.

When the drying temperature in the step (c) is less than 40°C, there is a problem that the drying is not performed effectively, and when the drying temperature in the step (c) is 200°C or higher, the catalyst may be deformed. Therefore, these ranges are not preferable.

Next, a method for regenerating a catalyst by using a hydrothermal hydrogenation reaction is provided. The hydrothermal hydrogenation reaction refers only to a hydrogenation reaction performed under high pressure hydrogen and solvent conditions at high temperature for regeneration of the catalyst, and should distinguish from a general hydrogenation reaction described in the present invention.

According to an embodiment of the present invention, the method for regenerating the catalyst may include the steps of: (i) adding a used catalyst and a solvent to a reactor; (ii) replacing a composition of gas inside the reactor with hydrogen; and (iii) reactivating the catalyst by performing a hydrothermal hydrogenation reaction while stirring under a condition in which a reactor internal temperature is 100-400°C and a hydrogen gas pressure is 1-20 MPa.

A deactivated catalyst and a solvent are added to the reactor. In this case, distilled water is provided as the solvent, and high purity water such as ion-exchanged water or deionized water (DIW) is provided as the distilled water. When the distilled water to be used contains impurities, the impurities may adhere to the catalyst, which may reduce the activity of the catalyst. When the solvent is 100 parts by weight, the catalyst may be included in an amount of 0.1-30 parts by weight, and preferably 2 parts by weight.

As the solvent used in the method for regenerating the catalyst by using the hydrothermal hydrogenation reaction, not only water, but also a solvent containing carboxylic acid, alcohol, and carboxylic acid ester material may be used. In this case, based on 100 parts by weight of the solvent, water may be included in an amount of 50-100 parts by weight and at least one of carboxylic acid, alcohol, and carboxylic acid ester may be included in an amount of 0-50 parts by weight, and preferably 10-20 parts by weight. The hydrothermal hydrogenation reaction under such conditions is advantageous in terms of process efficiency because it is not necessary to replace the reaction solution with water in the continuous process and the batch process.

Next, for the hydrothermal hydrogenation reaction, a method for replacing the composition of the gas inside the reactor with hydrogen is provided. After replacing all other gases in the reactor with hydrogen gas, the used catalyst is reactivated by performing the hydrothermal hydrogenation reaction while stirring at a temperature of 100-400°C and a hydrogen pressure of 1-20 Mpa. In this case, the temperature is preferably 200-300°C, and the hydrogen pressure is 3-15 MPa. In the above range, an effect of regenerating the catalyst in fouled by organic materials (ester). In this case, when the temperature is lower than 200°C, it is not preferably because the decomposition rate of the ester material causing fouling is not sufficient. When the temperature is higher than 300°C, it is not preferable because the catalyst may be deformed and the solvent used may be hydrogenated. In addition, when the hydrogen pressure is less than 3 MPa, it is not preferable because hydrogen participating in the reaction is not sufficiently present in the solvent. When the hydrogen pressure is greater than 15 MPa, there may be problems in process stability due to excessively high hydrogen pressure.

The reaction time of the hydrothermal hydrogenation reaction may be 0.5-48 hours, and preferably 12 hours. At this time, when the reaction time is less than 0.5 hours, it is not preferable because there is a possibility that the ester may not be decomposed sufficiently. When the reaction time is greater than 48 hours, it is not preferable in terms of process efficiency.

According to an embodiment of the present invention, the hydrogenation reaction, which uses the catalyst, converts a carboxylic acid functional group, a carboxylic acid ester functional group, an aldehyde functional group, or a ketone functional group into an alcohol functional group. More preferably, the catalyst for the hydrogenation reaction may be used to convert the dicarboxylic acid group of the reactant into the diol group of the product. In this case, the yield of the prepared dialcohol material may range from 85% to 100%, and the conversion rate of the dicarboxylic acid may range from 95% to 100%. In addition, the catalyst reactivated by the above-described method for regenerating the catalyst may also have an initial activity range value.

The carboxylic acid functional group may include one selected from the group consisting of oxalic acid, malonic acid, succinic acid, glutaric acid, adipic acid, pimelic acid, suberic acid, azelaic acid, sebacic acid, phthalic acid, isopthalic acid, cyclohexane dicarboxylic acid, and terephthalic acid, but the present invention is not limited thereto.

In addition, the hydrogenation reaction may be performed at a temperature of 150-300°C for 0.5-12 hours at a pressure of 50-150 bar. Preferably, the hydrogenation reaction of the CHDA may be performed at a temperature of 200-270°C and a pressure of 70-130 bar. At this time, when the temperature is lower than 150°C, it is not preferable because the hydrogenation reaction of the CHDA is not sufficiently activated. When the temperature is higher than 300°C, side reaction such as decomposition reaction of CHDA may occur. When the pressure is less than 50 bar, it is not preferable because hydrogen participating in the hydrogenation reaction of the CHDA is not sufficiently present in the solvent. When the pressure for the hydrogenation reaction is greater than 150 bar, a large amount of hydrogen is used, causing process stability problems. Most preferably, the pressure for the hydrogenation reaction may be 7-15 MPa, the reaction temperature may be 230-250°C, and the reaction time may be 0.5-6 hours. The hydrogenation reaction may be performed in various reactors. Preferably, the hydrogenation reaction may be performed in a continuous stirred tank reactor (CSTR) or a loop reactor.

According to an embodiment of the present invention, the activity of the used catalyst is lowered by fouling caused by deposition of a material produced in the process of converting the dicarboxylic acid group into the diol group. The produced material includes ester.

Under the above hydrogenation reaction conditions, an esterification reaction between a diol compound and a dicarboxylic acid compound occurs spontaneously. The resulting ester compound deposits on the surface of the catalyst during the hydrogenation reaction and causes fouling to deactivate the catalyst. Therefore, effectively removing the ester compound deposited on the hydrogenation catalyst after or during the reaction is essential to develop a catalytic process showing a long life.

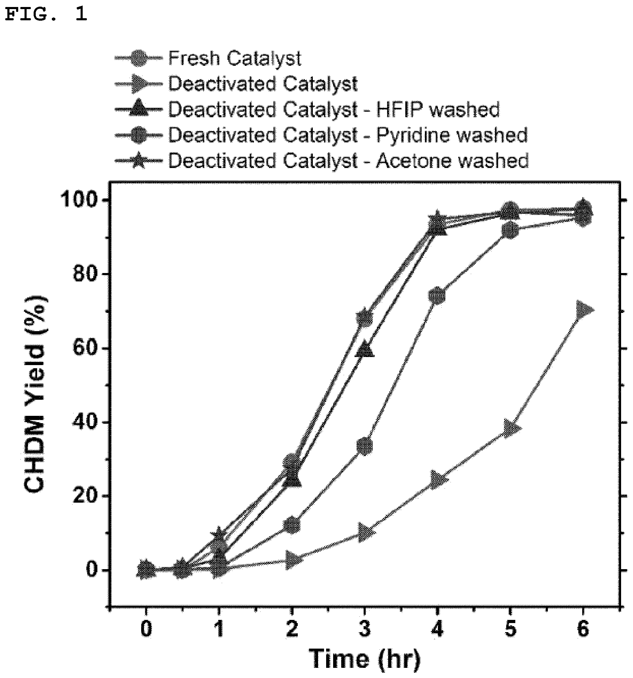

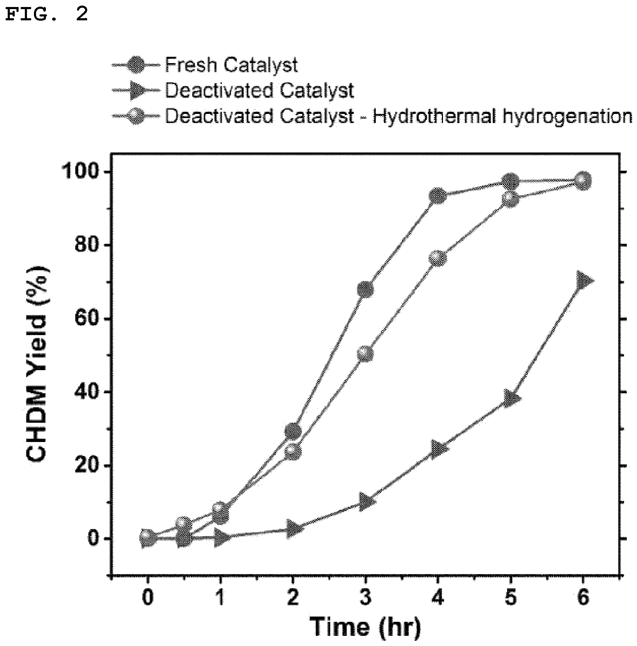

Therefore, deposits including the ester that is deposited on the surface of the catalyst and causes fouling may be removed using a method for regenerating a hydrogenation catalyst according to washing using an organic solvent and a hydrothermal hydrogenation reaction according to the present invention. The catalyst from which the deposit has been removed may be reactivated, and the catalyst may be regenerated close to the value of the initial activity. Results thereof are shown in FIGS. 1 and 2.

According to an embodiment of the present invention, the deactivated catalyst may include a catalyst in which a precious metal-transition metal is supported on a support. In this case, the precious metal may include one or more selected from the group consisting of palladium (Pd), rhodium (Rh), ruthenium (Ru), iridium (Ir), and platinum (Pt), and the transition metal may include one or more selected from the group consisting of tin (Sn), iron (Fe), rhenium (Re), and gallium (Ga), but the present invention is not limited thereto.

In addition, in the catalyst, ruthenium (Ru) may be provided as the precious metal, and tin (Sn) may be provided as the transition metal.

The support includes one or more selected from silica, alumina, zirconia, titania, and carbon. The carbon support is not particularly limited. At least one selected from the group consisting of activated carbon, carbon black, graphite, graphene, ordered mesoporous carbon (OMC), and carbon nanotubes may be used as the carbon support.

According to an embodiment of the present invention, the regenerated catalyst may be in the form of a powder, particles, or granules. Preferably, the catalyst is in the form of a powder. However, the present invention is not limited thereto.

Hereinafter, preferred examples are presented so as to help the understanding of the present invention. However, the following examples are for illustrative purposes only and the present invention is not limited by the following examples.

### <Examples>

### [Experimental Example 1] Cyclohexanedicarboxylic acid conversion experiment

A cyclohexanedicarboxylic acid conversion experiment was performed in a titania-lined stainless steel autoclave with a nominal volume of 250 ml and a maximum working pressure of 10.0 MPa.

At this time, 0.65 g of a catalyst, 2.44 g of a reactant (cyclohexanedicarboxylic acid), and 150 g of a solvent (H₂O) were added to a reactor. The reactor was pressurized to 90 bar by using hydrogen, and whether the reactor leaked was checked through a hydrogen detector. Oxygen inside the initial reactor was completely removed by depressurization and purging. Finally, after the internal pressure of the reactor was adjusted to 1 bar, the reactor was heated until the internal temperature of the reactor was 230°C, and hydrogen was pressurized to a pressure of 90 bar. The reaction was performed for 6 hours. At this time, the stirring was maintained at a rate of 1,000 rpm by using an overhead impeller. After the reaction, the reactor was cooled to room temperature and decompressed, such that the catalyst and the liquid product were separated by filtration and analyzed by gas chromatography with an HP-1 column.

### [Experimental Example 2] Analysis of deactivation cause of deactivated cyclohexanedicarboxylic acid hydrogenation catalyst

3 g of the hydrogenation catalyst deactivated by being used several times under the conditions of Experimental Example 1 was recovered, added to 300 ml of acetone, stirred at a rate of 300 rpm at room temperature for 1 hour, separated by filtration, and then recovered. After that, a filtered acetone washing solution was evaporated using a rotary evaporator to separate organic materials deposited on the catalyst. The separated organic materials were analyzed by gel permeation chromatography (GPC). Table 1 shows the types/ratios of organic materials deposited on the catalyst and the types/ratios of organic materials remaining in the reaction solution.

**[Table 1]**

| | Reaction solution | Deactivated catalyst washing solution |
|---|---|---|
| Light molecule A | 0 | 1.12 |
| Light molecule B | 3.04 | 20.75 |
| Cyclohexanedimethanol | 96.24 | 68.72 |
| Ester products | 0.72 | 9.41 |

### [Example 1] Washing of deactivated hydrogenation catalyst using organic solvent

3 g of a deactivated hydrogenation catalyst was added to 300 ml of an organic solvent (acetone, pyridine, and hexafluoroisopropanol), stirred at a rate of 300 rpm at room temperature for 1 hour, separated by filtration, and then recovered. The washing method was repeated three times. The finally recovered catalyst was dried at a temperature of 50°C for 12 hours in a vacuum state by using a vacuum oven. The catalysts washed as described above were denoted by "Deactivated Catalyst-Acetone washed", "Deactivated Catalyst-Pyridine washed", and "Deactivated Catalyst-HFIP washed". A cyclohexanedicarboxylic acid conversion experiment was performed on each catalyst washed using the organic solvent according to Experimental Example 1. Results thereof are shown in FIG. 1.

### [Example 2] Hydrothermal hydrogenation treatment of deactivated hydrogenation catalyst

A deactivated hydrogenation catalyst was added to a titania-lined stainless steel autoclave with a nominal volume of 250 ml and a maximum working pressure of 10.0 Mpa, and a hydrothermal hydrogenation treatment was performed thereon. At this time, 3g of the catalyst and 150 g of a solvent (H₂O) were added to the reactor. The reactor was pressurized to 90 bar by using hydrogen, and whether the reactor leaked was checked through a hydrogen detector. Oxygen inside the initial reactor was completely removed by depressurization and purging. Finally, after the internal pressure of the reactor was adjusted to 1 bar, the reactor was heated until the internal temperature of the reactor was 230°C, and hydrogen was pressurized to a pressure of 90 bar. The reaction was performed for 12 hours. At this time, the stirring was maintained at a rate of 1,000 rpm by using an overhead impeller. After the reaction was completed, the catalyst was separated and recovered by filtration. The catalyst on which the hydrothermal hydrogenation reaction has been performed as described above was denoted by "Deactivated Catalyst-Hydrothermal hydrogenation". A cyclohexanedicarboxylic acid conversion experiment was performed on the catalyst, on which the hydrothermal hydrogenation reaction has been performed, according to Experimental Example 1. Results thereof are shown in FIG. 2.

### [Comparative Example 1] Basic aqueous solution treatment of deactivated hydrogenation catalyst

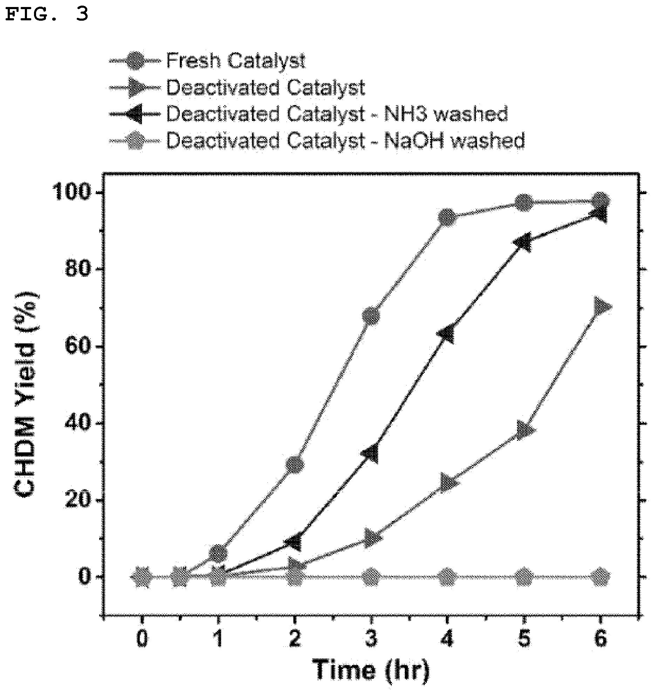

3 g of a deactivated hydrogenation catalyst was added to 300 ml of 1M basic aqueous solution (NaOH, NH₃), stirred at a rate of 300 rpm at room temperature for 1 hour, separated by filtration, and then recovered. The washing method was repeated three times. The finally recovered catalyst was dried at a temperature of 50°C for 12 hours in a vacuum state by using a vacuum oven. The catalysts washed as described above were denoted by "Deactivated Catalyst-NaOH washed" and "Deactivated Catalyst-NH₃ washed". A cyclohexanedicarboxylic acid conversion experiment was performed on each catalyst washed using the basic aqueous solution according to Experimental Example 1. Results thereof are shown in FIG. 3.

### [Comparative Example 2] Reduction treatment of deactivated hydrogenation catalyst

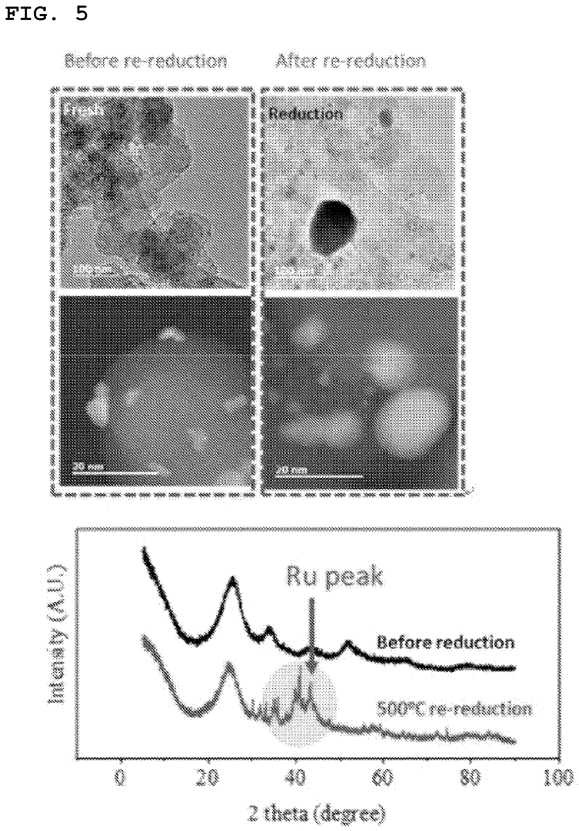

After 3 g of a deactivated hydrogenation catalyst was added to a tubular quartz reactor, hydrogen was reduced at a temperature of 500°C for 3 hours while flowing at a rate of 300 ml/min. At this time, a temperature increase rate was 5°C/min. After the reduction was completed, hydrogen was flowed at a rate of 300 ml/min and cooled to room temperature. After nitrogen was flowed at a rate of 300 ml/min for 30 minutes at room temperature, 5% oxygen/nitrogen mixed gas was flowed at a rate of 600 ml/min for 1 hour to passivate the catalyst. STEM and XRD analysis of the catalyst was performed. Results thereof are shown in FIG. 5.

As described above, as shown in Table 1, it can be confirmed that, when the deactivated hydrogenation catalyst is washed using the organic solvent, a larger amount of ester is present than the reaction solution. In this manner, it can be confirmed that the ester produced in the dicarboxylic acid to dialcohol conversion reaction continuously deposits on the surface of the catalyst to cause fouling of the catalyst, and thus the catalyst is deactivated.

As shown in FIG. 1, it can be confirmed that, when the deactivated hydrogenation catalyst is washed using the organic solvent (aceonte, pyridine, HFIP), the activity thereof is recovered to that substantially similar to the initial activity.

As shown in FIG. 2, it can be confirmed that, when the hydrothermal hydrogenation reaction is performed on the deactivated hydrogenation catalyst at a temperature of 230°C, the activity thereof is recovered to that substantially similar to the initial activity.

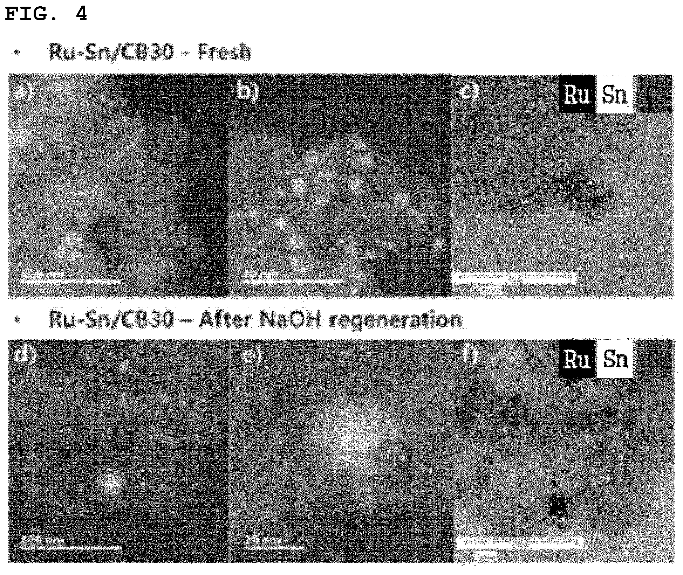

As shown in FIG. 3, when the deactivated hydrogenation catalyst is washed using 1M basic aqueous solution (NaOH, NH₃), the activity thereof worsens. The STEM-EDX analysis result is shown in FIG. 4. From the above results, it is determined that a significant part of the ruthenium (Ru)-tin (Sn)/tin (Sn) of the carbon catalyst was eluted during the washing using the basic aqueous solution. Therefore, it was confirmed that sodium stannate was formed according to [Reaction Formula 1] and [Reaction Formula 2] below.

[Reaction Formula 1 > Sn + 2NaOH + 4H₂O → Na₂[Sn(OH)₆] + 2H₂

[Reaction Formula 2] SnO₂ + 2NaOH + 2H₂O → Na₂[Sn(OH)₆]

It can be confirmed in FIG. 5 that even in the case of the catalyst re-reduced while flowing hydrogen at a temperature of 500°C, the activity thereof was further deteriorated, and the results of STEM and XRD analysis can also be confirmed in FIG. 6.

Although the present invention has been described with reference to the drawings according to embodiments of the present invention, it will be understood by those of ordinary skill in the art that various applications and modifications can be made thereto without departing from the scope of the present invention.

## Claims

1. A method for regenerating a catalyst used in a hydrogenation reaction of carboxylic acid or carboxylic acid ester, the method comprising the steps of:
(a) adding a used catalyst to an organic solvent and washing the catalyst while stirring the catalyst;
(b) separating and recovering the catalyst by filtering after the washing; and
(c) drying and reactivating the separated and recovered catalyst.

2. A method for regenerating a catalyst used in a hydrogenation reaction of carboxylic acid or carboxylic acid ester, the method comprising the steps of:
(i) adding a used catalyst and a solvent to a reactor;
(ii) replacing a composition of gas inside the reactor with hydrogen; and
(iii) reactivating the catalyst by performing a hydrothermal hydrogenation reaction while stirring under a condition in which a reactor internal temperature is 100-400°C and a hydrogen gas pressure is 1-20 Mpa.

3. The method of claim 1, wherein the organic solvent includes at least one selected from the group consisting of acetone, pyridine, hexafluoroisopropanol, methanol, ethanol, propanol, butanol, cyclohexane, toluene, and dichloromethane.

4. The method of claim 1, wherein the step (a) of washing the catalyst while stirring the catalyst is performed at 0-150°C for 0.15-12 hours.

5. The method of claim 1, wherein the drying in the step (c) is performed at a temperature of 40-200°C.

6. The method of claim 2, wherein the solvent in the step (i) includes at least one selected from water, carboxylic acid, alcohol, and carboxylic acid ester.

7. The method of claim 6, wherein, based on 100 parts by weight of the solvent, water is included in an amount of 50-100 parts by weight and at least one of carboxylic acid, alcohol, and carboxylic acid ester is included in an amount of 0-50 parts by weight.

8. The method of claim 2, wherein the hydrothermal hydrogenation reaction in the step (iii) is performed for 0.5-48 hours.

9. The method of claim 1 or 2, wherein the hydrogenation reaction converts a dicarboxylic acid group into a diol group.

10. The method of claim 1 or 2, wherein the carboxylic acid functional group includes one selected from the group consisting of oxalic acid, malonic acid, succinic acid, glutaric acid, adipic acid, pimelic acid, suberic acid, azelaic acid, sebacic acid, phthalic acid, isopthalic acid, cyclohexane dicarboxylic acid, and terephthalic acid.

11. The method of claim 1 or 2, wherein the activity of the used catalyst is lowered by fouling caused by deposition of a material produced in a process of converting a dicarboxylic acid group into a diol group.

12. The method of claim 11, wherein the produced material includes ester.

13. The method of claim 1 or 2, wherein the catalyst includes a precious metal-transition metal supported on a support.

14. The method of claim 13, wherein the precious metal includes at least one selected from the group consisting of palladium (Pd), rhodium (Rh), ruthenium (Ru), iridium (Ir), and platinum (Pt), and
the transition metal includes at least one selected from the group consisting of tin (Sn), iron (Fe), rhenium (Re), and gallium (Ga).

15. The method of claim 13, wherein the support includes at least one selected from silica, alumina, zirconia, titania, and carbon.

16. The method of claim 15, wherein the carbon is at least one selected from the group consisting of activated carbon, carbon black, graphite, graphene, ordered mesoporous carbon (OMC), and carbon nanotubes.
